# EUROPEAN PATENT SPECIFICATION

(11) **EP 3 900 792 B1**
(45) Date of publication and mention of the grant of the patent: **13.07.2022**
(21) Application number: 20170357.6
(22) Date of filing: 20.04.2020
(51) Int. Cl.: A61Q 7/00, A61K 8/49, A61P 17/14

(54) **TREATMENT REGIMEN WITH MINOXIDIL AND CAFFEINE**
BEHANDLUNGSSCHEMA MIT MINOXIDIL UND KOFFEIN
RÉGIME DE TRAITEMENT AU MINOXIDIL ET À LA CAFÉINE

(43) Date of publication of application: 27.10.2021
(73) Proprietor: Dr. Kurt Wolff GmbH & Co. KG, 33611 Bielefeld (DE)
(72) Inventor: SCHULZE ZUR WIESCHE, Erik., 33611 Bielefeld (DE); VOELKER, Joern Michael., 33611 Bielefeld (DE); BECKER, Maike., 33611 Bielefeld (DE)
(74) Representative: Ter Meer Steinmeister & Partner

(56) References cited:
- DE-A1-102005 003 949
- US-A- 5 006 332
- US-A1- 2016 220 465
- US-A1- 2019 343 835

## Description

### Field of the invention

The present invention is directed to a method for the treatment and/or prevention of hair loss comprising the application of minoxidil and caffeine.

### Background of the invention

Hair loss, or alopecia, constitutes a concern affecting all humans. Alopecia can be caused by hereditary factors, hormonal factors, surgery, trauma, medical treatments like chemotherapy, or stress.

Due to modern beauty ideals, the search for effective ways for treating or preventing hair loss is an ongoing endeavor.

Only few drugs have been clinically proven to be effective for the prevention of hair loss, such as minoxidil, finasteride, and dutasteride.

In this context, US2019/343835 A1 discloses a method for treating hair loss comprising the application to the hair and/or skin of a composition comprising both minoxidil and caffeine.

The exact mechanism of action of minoxidil is still not fully elucidated but it is believed that the metabolite minoxidil sulfate plays an important role in the proliferation of hair keratinocytes.

Minoxidil is commonly applied topically as an ointment to the scalp, i.e. in the morning and in the evening. Subsequently, the ointment is left in contact with the scalp so that the minoxidil can be absorbed through the skin (leave-on product). However, commercially available minoxidil solutions commonly contain propylene glycol, which usually results in oily hair that has a fatty and glossy appearance. In addition, dandruff and skin rashes are frequently observed side effects that are experienced during the treatment of hair loss.

Therefore, it was an object of the present invention to provide an administration regimen for the treatment of hair loss that overcomes the above-mentioned drawback while maintaining effectiveness of the treatment. In particular, it was an object of the present invention to provide an administration regimen that prevents fatty appearance of the hair while maintaining effectiveness. It was another object of the invention to provide an administration regimen for the treatment of hair loss that has less side effects while maintaining effectiveness.

In addition, it was a further object of the present invention to provide an administration regimen that results in a synergistic effect regarding the treatment and/or prevention of hair loss.

### Summary of the invention

These objects have surprisingly been solved by a non-therapeutic method for the treatment and/or prevention of hair loss comprising the following steps in any order:
(a) topically applying a composition (1) comprising minoxidil to the scalp and leaving the composition (1) in contact with the scalp for 5 to 15 h;
(b) topically applying a composition (2) comprising a surfactant and optionally caffeine to the scalp and leaving the composition (2) in contact with the scalp for 1 to 10 minutes; and at least in case when composition (2) does not comprise caffeine
(c) topically applying a composition (3) comprising caffeine to the scalp and leaving the composition (3) in contact with the scalp for at least 6 h.

It has surprisingly been found that according to the above aspect, minoxidil can be applied only once daily without sacrificing any of its effectiveness and at the same time fatty or glossy appearances of the hair is reduced due to the application of composition (2).

In another aspect, the present invention relates to a combination of a composition (1) comprising minoxidil and a composition (2) comprising caffeine for use in the treatment and/or prevention of hair loss,
wherein administration comprises the following steps in any order:
(a) topically applying a composition (1) comprising minoxidil to the scalp and leaving the composition (1) in contact with the scalp for 5 to 15 h; and
(b) topically applying a composition (2) comprising a surfactant and optionally caffeine to the scalp and leaving the composition (2) in contact with the scalp for 1 to 10 minutes; and at least in case when composition (2) does not comprise caffeine
(c) topically applying a composition (3) comprising caffeine to the scalp and leaving the composition (3) in contact with the scalp for at least 6 h.

The present invention also relates to a kit of parts comprising a composition (1) comprising minoxidil, a composition (2) comprising a surfactant and/or caffeine and at least in case when composition (2) does not comprise caffeine, a composition (3) comprising caffeine, and optionally a leaflet.

In yet another aspect, the invention relates to the use of the kit of parts for the cosmetic treatment and/or prevention of hair loss.

In another aspect, the intention relates to the kit of parts for use in the treatment and/or prevention of hair loss.

### Detailed description

The following definitions are relevant in connection with the embodiments of the present invention.

The meaning of the term "comprising" is to be interpreted as encompassing all the specifically mentioned features as well optional, additional, unspecified ones, whereas the term "consisting of" only includes those features as specified. Therefore, "comprising" includes as a limiting case the meaning of "consisting of".

The term "weight-%" or "wt.-%" is to be understood to refer to the weight amount of the component relative to the total weight amount of the respective composition, if not specified otherwise. The term "volume-%" or "vol.-%" is to be understood to refer to the volume amount of the component relative to the total volume amount of the respective composition, if not specified otherwise.

Preferred embodiments according to the invention are defined hereinafter. The preferred embodiments are preferred alone or in combination. Further, it is to be understood that the following preferred embodiments refer to all aspects of the present invention, i.e. the non-therapeutic method, the combination for use, the kit of parts, the use of the kit of parts and the kit of parts for use.

In an embodiment, the composition (1) comprises 0.1 to 5 wt.-% minoxidil. It is preferred that composition (1) comprises 1 to 5 wt.-% minoxidil. In a particularly preferred embodiment, the composition (1) comprises 2, 2.5, 3, 3.5, 4, 4.5 or 5 wt.- % minoxidil.

In an embodiment, the composition (1) is a gel, a foam or a solution, preferably a solution.

In case the composition (1) is a foam, composition (1) may comprise cetyl alcohol, stearyl alcohol, and butylated hydroxytoluene. In case composition (1) is a foam, it does typically not contain a glycol compound, such as propylene glycol.

In case the composition (1) is a solution, composition (1) may comprise an alcohol selected from the group consisting of ethanol, isopropanol, *n*-propanol, and mixtures thereof, and/or a glycol selected from the group consisting of propylene glycol, dipropylene glycol, 1,3-butylene glycol, pentylene glycol, dipentylene glycol, and mixtures thereof and/or water. It is preferred that the alcohol is ethanol and/or isopropanol. It is preferred that the glycol is propylene glycol. It is even more preferred that the composition (1) comprises 1 to 5 wt.-% minoxidil, ethanol, propylene glycol, and water.

In an embodiment, the composition (1) comprises 10 to 80 vol.-% ethanol, 20 to 50 vol.-% ethanol, or 25 to 35 vol.-% ethanol. It is preferred that the composition (1) comprises 25 to 35 vol.-% ethanol. In an embodiment, the composition (1) comprises 10 to 80 vol.-%, 20 to 70 vol.-% or 40 to 60 vol.-% propylene glycol. In a preferred embodiment, the composition (1) comprises 1 to 5 wt.-% minoxidil, 10 to 80 vol.-% propylene glycol and 10 to 80 vol.-% ethanol, and water.

In an embodiment, the composition (1), the composition (2), and/or the composition (3) comprises a preservative independently selected from the group consisting of benzoic acid, benzyl alcohol, chlorocresol, paraben esters, sorbic acid, ascorbic acid and salts thereof, sodium metabisulfite, chelating agents, citric acid, salicylic acid, and mixtures thereof.

In an embodiment, the composition (1), the composition (2), and/or the composition (3) comprises a pH modifier independently selected from the group consisting of diethanolamine, lactic acid, monoethanolamine, sodium hydroxide, sodium phosphate, triethanolamine, and mixtures thereof.

In an embodiment, the hair loss is hereditary hair loss. Hereditary hair loss commences after a certain age and may therefore also be called hair loss due to ageing. Androgenic alopecia, often also called androgenetic alopecia, is the most common hereditary hair loss and it was believed that it is caused by a high free concentration of androgens, such as DHT (dihydrotestosterone). Androgenic alopecia is gender-specific, resulting in male or female pattern hair loss. In another embodiment, the hair loss is alopecia areata, scarring alopecia, chemotherapyinduced alopecia, chronic telogen effluvium, and/or hair shaft disorder.

As the compositions (1) and (3) are left in contact with the scalp for an extended time period, they are also referred to as leave-on compositions in contrast to composition (2) which is a rinse-off composition, as it is typically rinsed off after 1 to 10 minutes. Composition (2) is commonly applied after the composition (1). The purpose of applying the composition (2) in step (b) is to remove or wash out the composition (1) from the hair and from the scalp.

In an embodiment, the composition (2) comprises a surfactant and 0.01 to 10 wt.-% of caffeine. It is preferred that the composition (2) is a shampoo. It is more preferred, that the composition (2) is a caffeine shampoo comprising 0.05 to 10, 0.1 to 5, or 0.2 to 1 wt.-% caffeine. In an embodiment, composition (3) comprises 0.05 to 10, 0.1 to 5, or 0.2 to 1 wt.-% caffeine.

The sequential treatment with minoxidil and caffeine is an essential part of the present invention and caffeine should be applied to the scalp either as part of applying composition (2) as rinse-off caffeine shampoo and/or as part of applying composition (3) as a leave-on caffeine lotion. In case the composition (2) does not contain caffeine, the step (c) is a mandatory, i.e. not an optional step. In contrast, step (c) may also be performed as an additional, optional step in case composition (2) contains caffeine.

In an embodiment, the composition (3) does not contain a surfactant. In another embodiment, the composition (2) has a viscosity of 2000 to 6000 mPa·s, whereas the composition (3) has a viscosity of 1 to 100 mPa·s.

The viscosity is measured at 20 °C and a shear rate of 10/s with a rheometer using DIN 53019-1:2008-09.

Thus composition (2) and composition (3) differ from each other in that composition (2) is a rinse-off product and composition (3) is a leave-on product, and in that composition (2) has a higher viscosity than composition (3).

In an embodiment, the composition (2) and/or the composition (3) comprise a further additional active ingredient independently selected from the group consisting of vitamins, flavonoids, terpenes, saccharides, plant extracts, and mixtures thereof.

Non-limiting examples of vitamins are niacin, biotin, vitamin E (also referred to as tocopherol), vitamin A, and vitamin C. Vitamins are antioxidants and can have various additional useful properties.

Non-limiting examples of flavonoids are apigenin, luteolin, tangeritin, quercetin, kaempferol, myricetin, fisetin, galangin, isorhamnetin, pachypodol, rhamnazin, pyranoflavonols, and furanoflavonols.

Non-limiting examples of terpenes are farnesol, menthol, limonen, squalene, camphor, pinene, eucalyptol, retinol, geraniol, linalool, carvone and thymol.

Non-limiting examples of saccharides are saccharides derived from starch, sorbitol and maltitol.

Plant-extracts can have multiple purposes and are i.a. useful as natural antioxidant, antiaging, emollient, anti-inflammatory, anti-irritant, and/or antimicrobial component. Non-limiting examples of plant extracts are plant oils, such as soy oil, sunflower oil, almond oil, grape oil, avocado oil, castor oil, glycine soja oil, jojoba oil and oils from *Areca catechu, Crocus sativus, Curcuma longa,* green tea, *Crataevea murula, Rosmarinus Officinalis,* buckwheat seeds, *Emblica officinale, Ginkgo biloba, Centella asiatica, Psoraliea corylifolia, Citrus limonum, Aloe Vera, Matricaria recutita, Thea viridis, Vitis vinifera, Daucus carota, Lycopersicon esculentum, Allium sativum and Hamamelis virginiana.*

In a preferred embodiment, the composition (2) comprises 0.01 to 2 wt.-% niacinamide. In another preferred embodiment, the composition (2) comprises 0.01 to 2 wt.-% biotin.

In another embodiment, the composition (2) comprises hydrolyzed wheat protein. In yet another embodiment, the composition (2) comprises an amino acid, preferably arginine or leucine.

In an embodiment, the surfactant in composition (2) is selected from the group consisting of non-ionic surfactants, anionic surfactants, cationic surfactants, amphoteric surfactants and mixtures thereof. In another embodiment, the composition (2) comprises 1 to 4 vol.-% of a non-ionic surfactant.

Surfactants are molecules that comprise a hydrophilic and a hydrophobic moiety at opposite ends. Suitable surfactants are described in "Cosmetic Formulation of Skin Care Products", Draelos et al., Taylor & Francis Group, 2006.

In an embodiment, the non-ionic surfactant is selected from the group consisting of C₄-C₂₆ fatty alcohol ethoxylates, alkylphenol ethoxylates, ethoxylated amines/amides, glycerol C₄-C₂₆ fatty acid amides, glycerol C₄-C₂₆ fatty acid esters, sorbitol fatty acid esters, and mixtures thereof. The prefix "Cₓ-C_{y}" denotes the number of carbon atoms of the respective functional group. Non-limiting examples of a C₄-C₂₆ fatty alcohol ethoxylates are pentaethylene glycol monododecyl ether (also referred to as C12E5), hexaethylene glycol monododecyl ether (C12E6), heptaethylene glycol monododecyl ether (C12E7), and octaethylene glycol monodecyl ether (C12E8). Non-limiting examples of alkylphenol ethoxylates are Triton X-100^{®} (octyl phenol ethoxylate) and Nonidet P-40^{®} (4-nonylphenol ethoxylate). Non-limiting examples of ethoxylated amines/amides are polyethoxylated tallow amine, cocamide monoethanolamine, and cocamide diethanolamine. Non-limiting examples of glycerol C₄-C₂₆ fatty acid esters are glycerol monostearate and glycerol monolaurate. Non-limiting examples of sorbitol fatty acid esters are sorbitan monolaurate, sorbitan monostearate and sorbitan tristearate.

Anionic surfactants dissociate in water to yield an anionic surfactant in water. Anionic surfactants useful herein include alkyl sulfates and alkyl ether sulfates. These materials have the respective formulae ROSO₃M and RO(C₂H₄O)ₓSO₃M wherein R is a C₄-C₂₆ alkyl or C₄-C₂₆ alkenyl, x is 1 to 10, and M is a water-soluble cation such as ammonium, sodium, potassium and triethanolamine. The alkyl ether sulfates useful in the present invention are condensation products of ethylene oxide and monohydric alcohols having about 4 to about 26 carbon atoms. Preferably, R is C₁₄-C₁₆ alkyl or C₁₄-C₁₈ alkenyl in both the alkyl and alkyl ether sulfates. The alcohols can be derived from fats, e.g., coconut oil or tallow, or can be synthetic. Lauryl alcohol and straight chain alcohols derived from coconut oil are preferred herein. Such alcohols are reacted with 1 to 10, and especially 3, molar proportions of ethylene oxide and the resulting mixture of molecular species, having, for example, an average of 3 moles of ethylene oxide per mole of alcohol, is sulfated and neutralized. Specific examples of alkyl ether sulfates of the present invention are sodium coconut alkyl trioxyethylene sulfate; lithium tallow alkyl trioxyethylene sulfate; sodium tallow alkyl hexaoxyethylene sulfate; and sodium lauryl ether sulfate. Additional examples of anionic surfactants which come within the terms of the present invention are the reaction product of fatty acids esterified with isethionic acid and neutralized with sodium hydroxide where, for example, the fatty acids are derived from coconut oil; sodium or potassium salts of fatty acid amides of methyl tauride in which the fatty acids, for example, are derived from coconut oil.

Cationic surfactants comprise primary, secondary tertiary amines, such as octenidine dihydrochloride, and quaternary ammonium salts, such as cetrimonium bromide, cetlypyridinium chloride, benzalkonium chloride, benzethonium chloride, dimethyldioctadecylammonium chloride and dioctadecyldimethylammonium bromide.

Amphoteric surfactants have an anionic and a cationic functional group. Examples of amphoteric surfactants are derivatives of aliphatic secondary and tertiary amines in which the aliphatic radical can be straight chain or branched and wherein one of the aliphatic substituents contains from about 8 to about 18 carbon atoms and one contains an anionic water solubilizing group, e.g., carboxy, sulfonate, sulfate, phosphate, or phosphonate. Specific examples of amphoteric surfactants are sodium 3-dodecylaminopropionate, sodium 3-dodecylaminopropane sulfonate, and *N*-alkyltaurines such as the one prepared by reacting dodecylamine with sodium isethionate. Particularly preferred amphoteric surfactants are betaines such as, for example, coco-amidopropyl betaines. The amphoteric surfactant component may be present in the composition in an amount ranging from about 5 to about 15% by weight, and preferably from about 9 to about 12% by weight, based on the weight of the composition.

In an embodiment, the composition (2) and/or the composition (3) comprise a diluent independently selected from the group consisting of water, glycerin, a glycol, sorbitol, ethanol, triacetin and mixtures thereof. In an embodiment, the composition (2) and the composition (3) do not contain a glycol.

In an embodiment, the kit of parts further comprises an application device for applying the composition (1), the composition (2) and/or the composition (3) to the scalp. The application device is suitable as a means for applying any one of the compositions. The application device may be an integral part of the container in which the composition is provided in or can be an external device, such as a suitable pipette or a contact applicator that is adapted to be attached to an opening of the container in which any one of the compositions is provided in. The application device can also be a pumpspray applicator. It is particularly preferred that the application device contains a means for determining the amount of the composition that is applied to the scalp.

The following formulation of a composition (2) and composition (3) are examples of a typical formulation:
In a typical example, the composition (2) comprising caffeine contains 4.0 wt.-% sodium lauryl sulfate, 5.0 wt.-% sodium laureth sulfate, 1.2 wt.-% sodium lauroyl glutamate, 1.0 wt.-% hydroxypropyltrimonium hydrolyzed wheat, 3.0 wt.-% disodium laureth sulfosuccinate, 0.3 wt.-% biotin, 0.2 wt.-% tocopherylacetate, 0.2 wt.-% citric acid, 1 wt.-% caffeine, 0.3 wt.-% fragrance, 0.2 wt.-% potassium sorbate, 0.5 % salicylic acid, 0.05 wt.-% niacinamide, and purified water.

In a typical example, the composition (3) contains 40.0 wt.-% ethanol, 0.05 wt.-% tocopheryl acetate, 0.3 wt.-% PEG-40 hydrogenated castor oil, 0.3 wt.-% caffeine, 0.2 wt.-% glycine soya oil, 0.3 wt.-% panthenol, 0.2 wt.-% fragrance, 0.1 wt.-% menthol, 0.05 wt.-% niacinamide, and purified water.

In an embodiment, the administration consists of the following steps in any order:
(a) topically applying a composition (1) comprising minoxidil to the scalp and leaving the composition (1) in contact with the scalp for 5 to 15 h;
(b) topically applying a composition (2) comprising a surfactant and optionally caffeine to the scalp and leaving the composition (2) in contact with the scalp for 1 to 10 minutes; and at least in case when composition (2) does not comprise caffeine
(c) topically applying a composition (3) comprising caffeine to the scalp and leaving the composition (3) in contact with the scalp for at least 6 h.

In an embodiment, the steps are performed in sequential, alphabetical order. It is preferred that step (a) is performed in the evening and step (b) and step (c) are performed the next morning. Thus, the steps (a) to (c) are performed once within 24 hours. It is preferred that the composition (1) comprising minoxidil is applied only once daily to the scalp (and not twice daily).

Thus, in a particularly preferred embodiment, the administration comprises
(a) topically applying a composition (1) comprising minoxidil to the scalp in the evening and leaving the composition (1) in contact with the scalp overnight; and
(b) topically applying a composition (2) comprising a surfactant and/or caffeine to the scalp in the morning and leaving the composition (2) in contact with the scalp for 1 to 10 minutes; and at least in case when composition (2) does not comprise caffeine
(c) topically applying a composition (3) comprising caffeine to the scalp and leaving the composition (3) in contact with the scalp for at least 6 h.

In an even more preferred embodiment, the administration comprises
(a) topically applying a composition (1) comprising minoxidil to the scalp in the evening and leaving the composition (1) in contact with the scalp overnight; and
(b) topically applying a composition (2) comprising 1 to 4 vol.-% of a non-ionic surfactant and 0.01 to 10 wt.-% of caffeine to the scalp in the morning and leaving the composition (2) in contact with the scalp for 1 to 10 minutes.

In an even more preferred embodiment, the administration comprises
(a) topically applying a composition (1) comprising minoxidil to the scalp in the evening and leaving the composition (1) in contact with the scalp overnight; and
(b) topically applying a composition (2) comprising 1 to 4 vol.-% of a non-ionic surfactant and not comprising any caffeine to the scalp in the morning and leaving the composition (2) in contact with the scalp for 1 to 10 minutes; and
(c) topically applying a composition (3) comprising 0.01 to 5 wt.-% caffeine to the scalp and leaving the composition (3) in contact with the scalp for at least 6 h.

Most preferably, the administration regimen of steps (a) to (c) in accordance with the present invention is conducted once daily (within 24 hours) over a non-limited period of time, preferably for at least several months. In other words, the administration regimen of steps (a) to (c) in accordance with the present invention is repeated every day for the total course of the intended and/or prescribed treatment of the hair and scalp with the ingredients minoxidil and caffeine. Using this administration regimen, minoxidil is applied only once daily which overcomes the disadvantages known from the prior art while the overall effect on hair growth is not reduced.

### Examples

### Example 1: Treatment with minoxidil solution and caffeine shampoo (rinse-off)

A minoxidil solution containing 5 wt.-% minoxidil, 30 vol.-% ethanol, 50 vol.-% propylene glycol and water was provided.

A composition (2) being a caffeine shampoo containing 4.0 wt.-% sodium lauryl sulfate, 5.0 wt.-% sodium laureth sulfate, 1.2 wt.-% sodium lauroyl glutamate, 1.0 wt.-% hydroxypropyltrimonium hydrolyzed wheat, 3.0 wt.-% disodium laureth sulfosuccinate, 0.3 wt.-% biotin, 0.2 wt.-% tocopherylacetate, 0.2 wt.-% citric acid, 1 wt.-% caffeine, 0.3 wt.-% fragrance, 0.2 wt.-% potassium sorbate, 0.5 % salicylic acid, 0.05 wt.-% niacinamide, and purified water was provided.

Subjects applied 1 mL of minoxidil solution in the evening and applied the caffeine shampoo of composition (2) to scalp on the next morning. The composition (2) was left in contact with the scalp for 1 to 10 minutes and subsequently rinsed off.

### Example 2: Treatment with minoxidil solution, surfactant shampoo (rinse off) and caffeine solution (leave-on)

The treatment in Example 2 was identical to that in Example 1, except that the composition (2) of Example 1 did not contain caffeine and after rinsing off the composition (2), a caffeine solution as composition (3) containing 40.0 wt.-% ethanol, 0.05 wt.-% tocopheryl acetate, 0.3 wt.-% PEG-40 hydrogenated castor oil, 0.3 wt.-% caffeine, 0.2 wt.-% glycine soya oil, 0.3 wt.-% panthenol, 0.2 wt.-% fragrance, 0.1 wt.-% menthol, 0.05 wt.-% niacinamide, and purified water was applied to the scalp and left in contact with the scalp until the evening.

### Comparatie Example 1: Treatment with minoxidil solution twice daily without caffeine

In Comparative Example 1, subjects applied 1 mL of the minoxidil solution of Example 1 in the evening and applied a shampoo of composition (2), being identical to that used in Example 1 but not containing caffeine, to scalp on the next morning. The composition (2) was left in contact with the scalp for 1 to 10 minutes and subsequently rinsed off. Subsequently 1 mL of the minoxidil solution was applied to the scalp and left in contact with the scalp until the evening.

Thus, the minoxidil solution was applied twice daily without applying a caffeine containing shampoo and/or caffeine solution.

### Comparative Example 2: Treatment with minoxidil solution further containing caffeine twice daily

In Comparative Example 1, subjects applied 1 mL of the minoxidil solution of Example 1 additionally containing 1 wt.-% caffeine in the evening and applied a shampoo of composition (2), being identical to that used in Example 1 but not containing caffeine, to scalp on the next morning. The composition (2) was left in contact with the scalp for 1 to 10 minutes and subsequently rinsed off. Subsequently, 1 mL of the minoxidil solution additionally containing 1 wt.-% caffeine was applied to the scalp and left in contact with the scalp until the evening.

Thus, a minoxidil solution further comprising caffeine was applied twice daily without applying a caffeine containing shampoo and/or caffeine solution.

The subjects treated according to Example 1 showed less fatty hair compared to subjects treated according to Comparative Example 1 or Comparative Example 2. After 3 months, no reduction in the prevention of hair loss could be observed between subjects treated according to Example 1 and subjects treated according to Comparative Example 1 or Comparative Example 2.

The subjects treated according to Example 2 showed less fatty hair compared to subject treated according to Comparative Example 1 or Comparative Example 2. After 3 months, no reduction in the prevention of hair loss could be observed between subjects treated according to Example 2 and subjects treated according to Comparative Example 1 or Comparative Example 3.

## Claims

1. A non-therapeutic method for the treatment and/or prevention of hair loss comprising the following steps in any order:
(a) topically applying a composition (1) comprising minoxidil to the scalp and leaving the composition (1) in contact with the scalp for 5 to 15 h;
(b) topically applying a composition (2) comprising a surfactant and optionally caffeine to the scalp and leaving the composition (2) in contact with the scalp for 1 to 10 minutes; and at least in case when composition (2) does not comprise caffeine
(c) topically applying a composition (3) comprising caffeine to the scalp and leaving the composition (3) in contact with the scalp for at least 6 h.

2. A combination of a composition (1) comprising minoxidil and a composition (2) comprising caffeine for use in the therapeutic treatment and/or prevention of hair loss, wherein administration comprises the following steps in any order:
(a) topically applying a composition (1) comprising minoxidil to the scalp and leaving the composition (1) in contact with the scalp for 5 to 15 h; and
(b) topically applying a composition (2) comprising a surfactant and optionally caffeine to the scalp and leaving the composition (2) in contact with the scalp for 1 to 10 minutes; and at least in case when composition (2) does not comprise caffeine
(c) topically applying a composition (3) comprising caffeine to the scalp and leaving the composition (3) in contact with the scalp for at least 6 h.

3. Kit of parts comprising a composition (1) comprising minoxidil, a composition (2) comprising a surfactant and/or caffeine and at least in case when composition (2) does not comprise caffeine, a composition (3) comprising caffeine, and optionally a leaflet.

4. Use of the kit of parts according to claim 3 for the non-therapeutic cosmetic treatment and/or non-therapeutic prevention of hair loss.

5. The kit of parts according to claim 3 for use in the therapeutic treatment and/or prevention of hair loss.

6. The method of claim 1, the combination for use of claim 2, the kit of parts of claim 3, the use of claim 4, the kit of parts for use of claim 5,
wherein the composition (1) comprises 0.1 to 5 wt.-% minoxidil.

7. The method of claim 1 or 6, the combination for use of claim 2 or 6, the kit of parts of claim 3 or 6, the use of claim 4 or 6, the kit of parts for use of claim 5 or 6, wherein the composition (1) is a gel, a foam or a solution, preferably a solution.

8. The method of any one of claims 1, 6 and 7, the combination for use of any one of claims 2, 6 and 7, the kit of parts of any one of claims 3, 6 and 7, the use of any one of claims 4, 6 and 7, the kit of parts for use of any one of claims 5, 6 and 7, wherein the composition (1) comprises an alcohol selected from the group consisting of ethanol, isopropanol, n-propanol, and mixtures thereof, and/or wherein the composition (1) comprises a glycol selected from the group consisting of propylene glycol, dipropylene glycol, 1,3-butylene glycol, pentylene glycol, dipentylene glycol, and mixtures thereof.

9. The method of any one of claims 1 and 6 to 8, the combination for use of any one of claims 2 and 6 to 8, the kit of parts of any one of claims 3 and 6 to 8, the use of any one of claims 4 and 6 to 8, the kit of parts for use of any one of claims 5 to 8, wherein the composition (1) comprises 1 to 20 vol.-% propylene glycol and 20 to 80 vol.-% ethanol.

10. The method of any one of claims 1 and 6 to 9, the combination for use of any one of claims 2 and 6 to 9, the kit of parts of any one of claims 3 and 6 to 9, the use of any one of claims 4 and 6 to 9, the kit of parts for use of any one of claims 5 to 9, wherein the composition (2) comprises 0.01 to 10 wt.-% of caffeine.

11. The method of any one of claims 1 and 6 to 10, the combination for use of any one of claims 2 and 6 to 10, the kit of parts of any one of claims 3 and 6 to 10, the use of any one of claims 4 and 6 to 10, the kit of parts for use of any one of claims 5 to 10,
wherein the composition (2) comprises a further additional active ingredient selected from the group consisting of vitamins, flavonoids, terpenes, saccharides, plant extracts, and mixtures thereof.

12. The method of any one of claims 1 and 6 to 11, the combination for use of any one of claims 2 and 6 to 11, the kit of parts of any one of claims 3 and 6 to 11, the use of any one of claims 4 and 6 to 11, the kit of parts for use of any one of claims 5 to 11,
wherein the composition (2) comprises 0.01 to 2 wt.-% niacinamide and/or 0.01 to 2 wt.-% biotin.

13. The method of any one of claims 1 and 6 to 12, the combination for use of any one of claims 2 and 6 to 12, the kit of parts of any one of claims 3 and 6 to 12, the use of any one of claims 4 and 6 to 12, the kit of parts for use of any one of claims 5 to 12,
wherein the composition (2) comprises 1 to 4 vol.-% of a non-ionic surfactant.

14. The method of any one of claims 1 and 6 to 13, the combination for use of any one of claims 2 and 6 to 13, the kit of parts of any one of claims 3 and 6 to 13, the use of any one of claims 4 and 6 to 13, the kit of parts for use of any one of claims 5 to 13,
wherein the composition (2) comprises a diluent selected from the group consisting of water, glycerin, a glycol, sorbitol, ethanol, triacetin and mixtures thereof.

15. The kit of parts of any one of claims 3 and 6 to 14, further comprising an application device for applying the composition (1), the composition (2) and/or the composition (3) to the scalp.

## Patentansprüche

1. Nicht-therapeutisches Verfahren zur Behandlung und/oder Vorbeugung von Haarausfall umfassend die folgenden Schritte in beliebiger Reihenfolge:
(a) topisches Auftragen einer Zusammensetzung (1), die Minoxidil enthält, auf die Kopfhaut und Belassen der Zusammensetzung (1) in Kontakt mit der Kopfhaut für 5 bis 15 h;
(b) topisches Auftragen einer Zusammensetzung (2), die ein Tensid und gegebenenfalls Koffein enthält, auf die Kopfhaut und Belassen der Zusammensetzung (2) in Kontakt mit der Kopfhaut für 1 bis 10 Minuten; und zumindest in dem Fall, wenn die Zusammensetzung (2) kein Koffein enthält,
(c) topisches Auftragen einer Zusammensetzung (3), die Koffein enthält, auf die Kopfhaut und Belassen der Zusammensetzung (3) in Kontakt mit der Kopfhaut für mindestens 6 h.

2. Kombination aus einer Zusammensetzung (1), die Minoxidil enthält, und einer Zusammensetzung (2), die Koffein enthält, zur Verwendung bei der therapeutischen Behandlung und/oder Vorbeugung von Haarausfall, wobei die Verabreichung die folgenden Schritte in beliebiger Reihenfolge umfasst:
(a) topisches Auftragen einer Zusammensetzung (1), die Minoxidil enthält, auf die Kopfhaut und Belassen der Zusammensetzung (1) in Kontakt mit der Kopfhaut für 5 bis 15 h; und
(b) topisches Auftragen einer Zusammensetzung (2), die ein Tensid und gegebenenfalls Koffein enthält, auf die Kopfhaut und Belassen der Zusammensetzung (2) in Kontakt mit der Kopfhaut für 1 bis 10 Minuten; und zumindest in dem Fall, wenn die Zusammensetzung (2) kein Koffein enthält,
(c) topisches Auftragen einer Zusammensetzung (3), die Koffein enthält, auf die Kopfhaut und Belassen der Zusammensetzung (3) in Kontakt mit der Kopfhaut für mindestens 6 h.

3. Kit, umfassend eine Zusammensetzung (1), die Minoxidil enthält, eine Zusammensetzung (2), die ein Tensid und/oder Koffein enthält, und zumindest in dem Fall, wenn die Zusammensetzung (2) kein Koffein enthält, eine Zusammensetzung (3), die Koffein enthält, und gegebenenfalls eine Packungsbeilage.

4. Verwendung des Kits nach Anspruch 3 zur nicht-therapeutischen kosmetischen Behandlung und/oder nicht-therapeutischen Vorbeugung von Haarausfall.

5. Kit nach Anspruch 3 zur Verwendung bei der therapeutischen Behandlung und/oder Vorbeugung von Haarausfall.

6. Verfahren nach Anspruch 1, Kombination zur Verwendung nach Anspruch 2, Kit nach Anspruch 3, Verwendung nach Anspruch 4, Kit zur Verwendung nach Anspruch 5, wobei die Zusammensetzung (1) 0,1 bis 5 Gew. -% Minoxidil enthält.

7. Verfahren nach Anspruch 1 oder 6, Kombination zur Verwendung nach Anspruch 2 oder 6, Kit nach Anspruch 3 oder 6, Verwendung nach Anspruch 4 oder 6, Kit zur Verwendung nach Anspruch 5 oder 6, wobei die Zusammensetzung (1) ein Gel, ein Schaum oder eine Lösung, bevorzugt eine Lösung ist.

8. Verfahren nach einem der Ansprüche 1, 6 und 7, Kombination zur Verwendung nach einem der Ansprüche 2, 6 und 7, Kit nach einem der Ansprüche 3, 6 und 7, Verwendung nach einem der Ansprüche 4, 6 und 7, Kit zur Verwendung nach einem der Ansprüche 5, 6 und 7, wobei die Zusammensetzung (1) einen Alkohol enthält, ausgewählt aus der Gruppe bestehend aus Ethanol, Isopropanol, n-Propanol und Mischungen davon, und/oder wobei die Zusammensetzung (1) ein Glykol enthält, ausgewählt aus der Gruppe bestehend aus Propylenglykol, Dipropylenglykol, 1,3-Butylenglykol, Pentylenglykol, Dipentylenglykol und Mischungen davon.

9. Verfahren nach einem der Ansprüche 1 und 6 bis 8, Kombination zur Verwendung nach einem der Ansprüche 2 und 6 bis 8, Kit nach einem der Ansprüche 3 und 6 bis 8, Verwendung nach einem der Ansprüche 4 und 6 bis 8, Kit zur Verwendung nach einem der Ansprüche 5 bis 8, wobei die Zusammensetzung (1) 1 bis 20 Vol.-% Propylenglykol und 20 bis 80 Vol.-% Ethanol enthält.

10. Verfahren nach einem der Ansprüche 1 und 6 bis 9, Kombination zur Verwendung nach einem der Ansprüche 2 und 6 bis 9, Kit nach einem der Ansprüche 3 und 6 bis 9, Verwendung nach einem der Ansprüche 4 und 6 bis 9, Kit zur Verwendung nach einem der Ansprüche 5 bis 9, wobei die Zusammensetzung (2) 0,01 bis 10 Gew.-% Koffein enthält.

11. Verfahren nach einem der Ansprüche 1 und 6 bis 10, Kombination zur Verwendung nach einem der Ansprüche 2 und 6 bis 10, Kit nach einem der Ansprüche 3 und 6 bis 10, Verwendung nach einem der Ansprüche 4 und 6 bis 10, Kit zur Verwendung nach einem der Ansprüche 5 bis 10, wobei die Zusammensetzung (2) einen weiteren zusätzlichen Wirkstoff enthält, ausgewählt aus der Gruppe bestehend aus Vitaminen, Flavonoiden, Terpenen, Sacchariden, Pflanzenextrakten und Mischungen davon.

12. Verfahren nach einem der Ansprüche 1 und 6 bis 11, Kombination zur Verwendung nach einem der Ansprüche 2 und 6 bis 11, Kit nach einem der Ansprüche 3 und 6 bis 11, Verwendung nach einem der Ansprüche 4 und 6 bis 11, Kit zur Verwendung nach einem der Ansprüche 5 bis 11, wobei die Zusammensetzung (2) 0,01 bis 2 Gew.-% Niacinamid und/oder 0,01 bis 2 Gew.-% Biotin enthält.

13. Verfahren nach einem der Ansprüche 1 und 6 bis 12, Kombination zur Verwendung nach einem der Ansprüche 2 und 6 bis 12, Kit nach einem der Ansprüche 3 und 6 bis 12, Verwendung nach einem der Ansprüche 4 und 6 bis 12, Kit zur Verwendung nach einem der Ansprüche 5 bis 12, wobei die Zusammensetzung (2) 1 bis 4 Vol.-% eines nichtionischen Tensids enthält.

14. Verfahren nach einem der Ansprüche 1 und 6 bis 13, Kombination zur Verwendung nach einem der Ansprüche 2 und 6 bis 13, Kit nach einem der Ansprüche 3 und 6 bis 13, Verwendung nach einem der Ansprüche 4 und 6 bis 13, Kit zur Verwendung nach einem der Ansprüche 5 bis 13, wobei die Zusammensetzung (2) ein Verdünnungsmittel enthält, ausgewählt aus der Gruppe bestehend aus Wasser, Glycerin, einem Glykol, Sorbitol, Ethanol, Triacetin und Mischungen davon.

15. Kit nach einem der Ansprüche 3 und 6 bis 14, ferner umfassend eine Auftragvorrichtung zum Auftragen der Zusammensetzung (1), der Zusammensetzung (2) und/oder der Zusammensetzung (3) auf die Kopfhaut.

## Revendications

1. Procédé non thérapeutique pour le traitement et/ou la prévention de la chute de cheveux, comportant les étapes suivantes, dans un ordre quelconque, consistant à :
(a) appliquer par voie topique une composition (1) comportant du minoxidil sur le cuir chevelu et laisser la composition (1) en contact avec le cuir chevelu pendant 5 à 15 h ;
(b) appliquer par voie topique une composition (2) comportant un tensioactif et facultativement de la caféine sur le cuir chevelu et laisser la composition (2) en contact avec le cuir chevelu pendant 1 à 10 minutes ; et au moins dans le cas où la composition (2) ne comporte pas de caféine
(c) appliquer par voie topique une composition (3) comportant de la caféine sur le cuir chevelu et laisser la composition (3) en contact avec le cuir chevelu pendant au moins 6 h.

2. Combinaison d'une composition (1) comportant du minoxidil et d'une composition (2) comportant de la caféine pour une utilisation dans le traitement thérapeutique et/ou la prévention de la chute de cheveux,
dans laquelle l'administration comporte les étapes suivantes, dans un ordre quelconque, consistant à :
(a) appliquer par voie topique une composition (1) comportant du minoxidil sur le cuir chevelu et laisser la composition (1) en contact avec le cuir chevelu pendant 5 à 15 h ; et
(b) appliquer par voie topique une composition (2) comportant un tensioactif et facultativement de la caféine sur le cuir chevelu et laisser la composition (2) en contact avec le cuir chevelu pendant 1 à 10 minutes ; et au moins dans le cas où la composition (2) ne comporte pas de caféine
(c) appliquer par voie topique une composition (3) comportant de la caféine sur le cuir chevelu et laisser la composition (3) en contact avec le cuir chevelu pendant au moins 6 h.

3. Kit de parties comportant une composition (1) comportant du minoxidil, une composition (2) comportant un tensioactif et/ou de la caféine et au moins dans un cas où la composition (2) ne comporte pas de caféine, une composition (3) comportant de la caféine, et facultativement un feuillet.

4. Utilisation du kit de parties selon la revendication 3 pour le traitement cosmétique non thérapeutique et/ou la prévention non thérapeutique de la perte de cheveux.

5. Kit de parties selon la revendication 3 pour une utilisation dans le traitement thérapeutique et/ou la prévention thérapeutique de la perte de cheveux.

6. Procédé selon la revendication 1, combinaison pour une utilisation selon la revendication 2, kit de parties selon la revendication 3, utilisation selon la revendication 4, kit de parties pour une utilisation selon la revendication 5,
dans lesquels la composition (1) comporte de 0,1 à 5 % en poids de minoxidil.

7. Procédé selon la revendication 1 ou 6, combinaison pour une utilisation selon la revendication 2 ou 6, kit de parties selon la revendication 3 ou 6, utilisation selon la revendication 4 ou 6, kit de parties pour une utilisation selon la revendication 5 ou 6,
dans lesquels la composition (1) est un gel, une mousse ou une solution, de préférence une solution.

8. Procédé selon l'une quelconque des revendications 1, 6 et 7, combinaison pour une utilisation selon l'une quelconque des revendications 2, 6 et 7, kit de parties selon l'une quelconque des revendications 3, 6 et 7, utilisation selon l'une quelconque des revendications 4, 6 et 7, kit de parties pour une utilisation selon l'une quelconque des revendications 5, 6 et 7,
dans lesquels la composition (1) comporte un alcool choisi parmi le groupe constitué d'éthanol, d'isopropanol, de n-propanol et de mélanges de ceux-ci, et/ou
dans lesquels la composition (1) comporte un glycol choisi parmi le groupe constitué de propylène glycol, de dipropylène glycol, de 1,3-butylène glycol, de pentylène glycol, de dipentylène glycol et de mélanges de ceux-ci.

9. Procédé selon l'une quelconque des revendications 1 et 6 à 8, combinaison pour une utilisation selon l'une quelconque des revendications 2 et 6 à 8, kit de parties selon l'une quelconque des revendications 3 et 6 à 8, utilisation selon l'une quelconque des revendications 4 et 6 à 8, kit de parties pour une utilisation selon l'une quelconque des revendications 5 à 8,
dans lesquels la composition (1) comporte de 1 à 20 % en volume de propylène glycol et de 20 à 80 % en volume d'éthanol.

10. Procédé selon l'une quelconque des revendications 1 et 6 à 9, combinaison pour une utilisation selon l'une quelconque des revendications 2 et 6 à 9, kit de parties selon l'une quelconque des revendications 3 et 6 à 9, utilisation selon l'une quelconque des revendications 4 et 6 à 9, kit de parties pour une utilisation selon l'une quelconque des revendications 5 à 9, dans lesquels la composition (2) comporte de 0,01 % à 10 % en poids de caféine.

11. Procédé selon l'une quelconque des revendications 1 et 6 à 10, combinaison pour une utilisation selon l'une quelconque des revendications 2 et 6 à 10, kit de parties selon l'une quelconque des revendications 3 et 6 à 10, utilisation selon l'une quelconque des revendications 4 et 6 à 10, kit de parties pour une utilisation selon l'une quelconque des revendications 5 à 10,
dans lesquels la composition (2) comporte un ingrédient actif additionnel supplémentaire choisi parmi le groupe constitué de vitamines, de flavonoïdes, de terpènes, de saccharides, d'extraits de plantes et de mélanges de ceux-ci.

12. Procédé selon l'une quelconque des revendications 1 et 6 à 11, combinaison pour une utilisation selon l'une quelconque des revendications 2 et 6 à 11, kit de parties selon l'une quelconque des revendications 3 et 6 à 11, utilisation selon l'une quelconque des revendications 4 et 6 à 11, kit de parties pour une utilisation selon l'une quelconque des revendications 5 à 11,
dans lesquels la composition (2) comporte de 0,01 à 2 % en poids de niacinamide et/ou de 0,01 à 2 % en poids de biotine.

13. Procédé selon l'une quelconque des revendications 1 et 6 à 12, combinaison pour une utilisation selon l'une quelconque des revendications 2 et 6 à 12, kit de parties selon l'une quelconque des revendications 3 et 6 à 12, utilisation selon l'une quelconque des revendications 4 et 6 à 12, kit de parties pour une utilisation selon l'une quelconque des revendications 5 à 12,
dans lesquels la composition (2) comporte de 1 à 4 % en volume d'un tensioactif non ionique.

14. Procédé selon l'une quelconque des revendications 1 et 6 à 13, combinaison pour une utilisation selon l'une quelconque des revendications 2 et 6 à 13, kit de parties selon l'une quelconque des revendications 3 et 6 à 13, utilisation selon l'une quelconque des revendications 4 et 6 à 13, kit de parties pour une utilisation selon l'une quelconque des revendications 5 à 13,
dans lesquels la composition (2) comporte un diluant choisi parmi le groupe constitué d'eau, de glycérine, d'un glycol, de sorbitol, d'éthanol, de triacétine et de mélanges de ceux-ci.

15. Kit de parties selon l'une quelconque des revendications 3 et 6 à 14, comportant en outre un dispositif d'application pour appliquer la composition (1), la composition (2) et/ou la composition (3) sur le cuir chevelu.
